# EUROPEAN PATENT APPLICATION

(11) **EP 3 435 291 A1**
(43) Date of publication of application: **30.01.2019**
(21) Application number: 17785862.8
(22) Date of filing: 11.04.2017
(51) Int. Cl.: G06N 3/00

(54) **EMOTION DETERMINATION SYSTEM, SYSTEM, AND PROGRAM**

(30) Priority: 19.04.2016 JP 2016084031
(71) Applicant: Cocoro SB Corp., Tokyo 105-7309 (JP)
(72) Inventor: SON Masayoshi, Tokyo 105-7309 (JP); TSUTSUI Takashi, Tokyo 105-7309 (JP); TOMONAGA Kosuke, Tokyo 105-7309 (JP); OURA Kiyoshi, Tokyo 105-7309 (JP)
(74) Representative: Michalski Hüttermann & Partner Patentanwälte mbB
(86) International application number: PCT/JP2017/014873
(87) International publication number: WO 2017/183523

(57) **Abstract**

An emotion determining system includes: an input information generating unit to generate, based on detection signals of one or more sensors that are provided to a target object, input information for determining an emotion of the target object; a secretion information generating unit to generate, based on the detection signals, secretion information indicating secretion amounts of one or more endocrine substances; a parameter adjusting unit to adjust an operation parameter for determining the emotion from the input information based on the secretion amounts of the one or more endocrine substances indicated by the secretion information.

## Description

### BACKGROUND

### 1. TECHNICAL FIELD

The present invention relates to an emotion determining system, a system, and a program.

### 2. RELATED ART

A terminal that learns conversations between a user and his/her communication partner and accumulates, in a reply table, replies from the communication partner to questions from the user has been known (refer to Patent Document 1, for example). Also, an emotion generating apparatus including a neural net that receives an input of user information, equipment information and a current emotional state of a user him/herself to output a next emotional state has been known (refer to Patent Document 2, for example). Also, a technique to store spatiotemporal patterns in an associative memory including a plurality of electronic neurons that have a layer-neural net relationship having directive artificial synapse connectivity has been known (refer to Patent Document 3, for example).

### [Patent document]

Patent document 1: Japanese Patent Application Publication No. 2011-253389
Patent document 2: Japanese Patent Application Publication No. H10-254592
Patent document 3: Japanese Translation of PCT International Patent Application No. 2013-535067

Conventionally, there are problems that a variety of emotions cannot be generated of information detected by sensors.

### [General Disclosure]

The first aspect of the present invention provides an emotion determining system. The emotion determining system may include an input information generating unit to generate, based on detection signals of one or more sensors that are provided to a target object, input information for determining an emotion of the target object. The emotion determining system may include a secretion information generating unit to generate, based on the detection signals, secretion information indicating secretion amounts of one or more endocrine substances. The emotion determining system may include a parameter adjusting unit to adjust an operation parameter for determining the emotion from the input information based on the secretion amounts of the endocrine substances indicated by the secretion information.

The emotion determining system may include a first correspondence information storage unit to store first correspondence information that associates a plurality of the sensors with the secretion amounts of the endocrine substances respectively. The emotion determining system may change the secretion amounts of the endocrine substances associated with the respective sensors by the first correspondence information according to measurement values indicated by the detection signals of the plurality of sensors.

The emotion determining system may include an emotion determining unit to determine the emotion from the input information using the operation parameter.

The emotion determining unit may determine the emotion using a neural network that receives the input information as an input.

The operation parameter may be a coupling coefficient of an artificial synapse that is included in the neural network.

The emotion determining system may include a second correspondence information storage unit to store second correspondence information that associates each of a plurality of the artificial synapses with a corresponding one of the endocrine substances. The parameter adjusting unit may change each of the coupling coefficients of the plurality of artificial synapses associated with the endocrine substances by the second correspondence information according to the secretion amounts of the endocrine substances indicated by the secretion information.

The input information generating unit may acquire measurement values indicated by the detection signals at predetermined frequency and generate an input value that is a predetermined value for the neural network. The secretion information generating unit may change the secretion amounts of the endocrine substances according to the measurement values.

The input information generating unit may generate input values that are predetermined values for the neural network at the frequencies that are different for a plurality of the sensors.

The neural network may include a plurality of emotion artificial neurons that are artificial neurons for which emotions are determined.

The emotion determining unit may determine the current emotion based on respective current firing states of the plurality of emotion artificial neurons.

The secretion information generating unit may change the secretion amounts of the endocrine substances according to change amounts over time of measurement values indicated by the detection signals.

The emotion determining system may include a control unit to control the target object according to the emotion.

The second aspect of the present invention provides a system. The system may include the emotion determining system described above and the target object.

The third aspect of the present invention provides a program to make a computer function as the emotion determining system described above.

The summary clause does not necessarily describe all necessary features of the embodiments of the present invention. The present invention may also be a sub-combination of the features described above.

### BRIEF DESCRIPTION OF THE DRAWINGS

Fig. 1 schematically shows a configuration of a vehicle 10 according to one embodiment.
Fig. 2 schematically shows a block configuration of an emotion determining system 100, together with a recording medium 290.
Fig. 3 schematically shows an emotion map 300.
Fig. 4 schematically shows a part of a neural network that is used for the emotion determining system 100.
Fig. 5 is exemplary sensor correspondence information associating the accelerator opening with the amounts of the endocrine substances.
Fig. 6 is exemplary sensor correspondence information associating the roll angle with the endocrine substance.
Fig. 7 is exemplary coupling coefficient correspondence information associating the noradrenaline amount with the coupling coefficients BS.
Fig. 8 is a flowchart illustrating operations of the units in the emotion determining system 100.
Fig. 9 schematically shows information output from an UI unit 180.

### DESCRIPTION OF EXEMPLARY EMBODIMENTS

Hereinafter, (some) embodiment(s) of the present invention will be described. The embodiment(s) do(es) not limit the invention according to the claims, and all the combinations of the features described in the embodiment(s) are not necessarily essential to means provided by aspects of the invention.

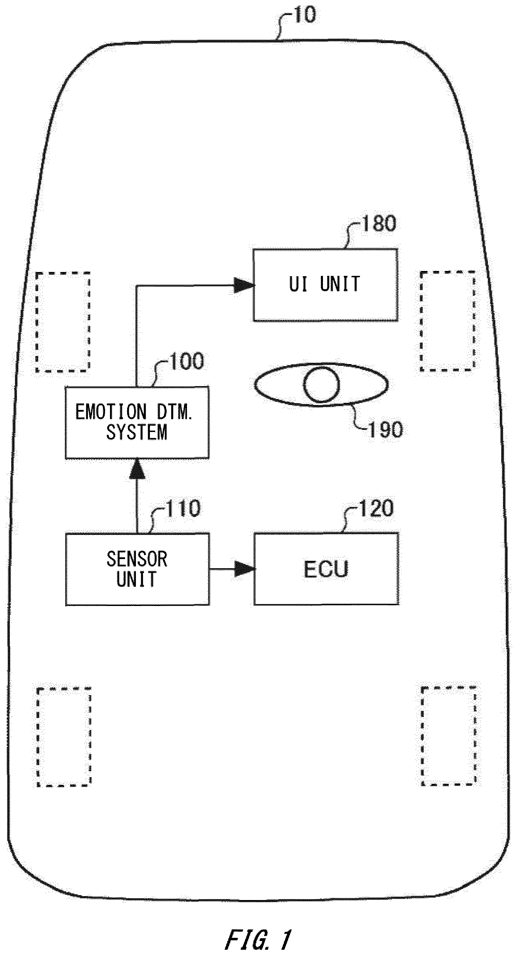

Fig. 1 schematically shows a configuration of a vehicle 10 according to one embodiment. The vehicle 10 includes an emotion determining system 100, a sensor unit 110, an electronic control unit (ECU) 120, an UI unit 180 to provide a user interface (UI) between the vehicle 10 and the occupant. Note that the vehicle 10 is an automobile. The vehicle 10 is an exemplary target object of the emotion determining system 100.

The sensor unit 110 has a plurality of sensors to detect states of the units of the vehicle 10. For example, the sensor unit 110 may include: a wheel speed sensor to detect the rotational speed of at least one of the front and rear wheels; an accelerator opening sensor to detect step-in quantity of the accelerator device by the driver; a throttle opening sensor to detect the throttle valve opening; an engine rotation speed sensor to detect the rotation speed of the engine or motor as the prime mover; an output rotating speed sensor to detect the output rotating speed of the transmission; a front-rear acceleration sensor to detect acceleration in the front-rear direction; a lateral acceleration sensor to detect acceleration in the right-left direction that is approximately orthogonal to the front-rear direction; a yaw rate sensor to detect the changing speed of the rotation angle in the turning direction; a steering angle sensor to detect steering quantity of the steering device by the driver; a brake sensor to detect step-in quantity of the braking pedal by the driver; a remaining amount sensor to detect a remaining amount of the fuel or battery, or the like. The respective sensors described above included in the sensor unit 110 output detection signals to the ECU 120 that is an electronic control apparatus. The electronic control apparatus 120 performs operations based on the acquired detection signals and outputs the operation results to each unit of the vehicle 10 as control command signals.

The various sensors described above included in the sensor unit 110 output the detection signals to the emotion determining system 100. Note that the emotion determining system 100 may acquire the detection signals from the ECU 120 via CAN or the like. The emotion determining system 100 performs operations based on the acquired detection signals and determines an emotion to assign to the vehicle 10. The emotion determining system 100 controls the vehicle 10 based on the determined emotion. For example, the emotion determining system 100 displays a face icon to express the determined emotion on the UI unit 180. Also, the emotion determining system 100 may output a corresponding tone of voice to the emotion from the UI unit 180, and have a conversation with the user 190 who is the driver of the vehicle 10.

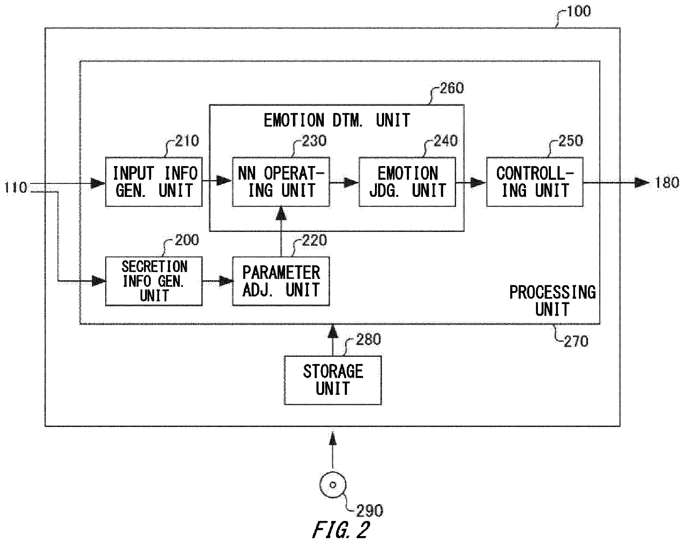

Fig. 2 schematically shows a block configuration of the emotion determining system 100, together with a recording medium 290. The emotion determining system 100 includes a processing unit 270, and a storage unit 280. The processing unit 270 includes a secretion information generating unit 200, an input information generating unit 210, a parameter adjusting unit 220, an emotion determining unit 260, and a control unit 250. The emotion determining unit 260 has an NN operating unit 230 and an emotion judging unit 240.

The input information generating unit 210 generates input information for determining the emotion of the vehicle 10 based on the detection signals of the one or more sensors provided in the vehicle 10. For example, the input information generating unit 210 generates input information for determining the emotion of the vehicle 10 based on the detection signals of the sensors included in the sensor unit 110.

The secretion information generating unit 200 generates secretion information indicating secretion amounts of one or more endocrine substances based on the detection signals of the one or more sensors provided in the vehicle 10. For example, the secretion information generating unit 200 generates secretion information indicating secretion amounts of the one or more endocrine substances based on the detection signals of the respective sensors included in the sensor unit 110. The endocrine substances can include noradrenaline, dopamine, CRH (corticotropin releasing hormone), and the like. Note that the secretion information indicating secretion amounts of the endocrine substances are pseudoly generated as the internal information in the emotion determining system 100, and no endocrine substance is actually secreted. Note that the secretion information generating unit 200 may change the secretion amounts of the endocrine substances according to change amounts over time of measurement values indicated by the detection signals.

The parameter adjusting unit 220 adjusts operation parameters for determining the emotion from the input information based on the secretion amounts of the endocrine substances indicated by the secretion information generated by the secretion information generating unit 200. Then, the emotion determining unit 260 determines the emotion from the input information, using the operation parameters.

The storage unit 280 stores correspondence information to associate the plurality of respective sensors included in the sensor unit 110 with the secretion amounts of the endocrine substances. The secretion information generating unit 200 changes the secretion amounts of the endocrine substances associated with the respective sensors by the correspondence information, according to the measurement values indicated by the respective detection signals of the plurality of sensors.

Note that the emotion determining unit 260 determines the emotion using a neural network (NN) that receives the input information as an input. Specifically, it determines the emotion by the NN operating unit 230 performing operations of the neural network and the emotion judging unit 240 judging the emotion based on the operation result of the neural network by the NN operating unit 230. Here, the operation parameters adjusted by the parameter adjusting unit 220 may be coupling coefficients of artificial synapses included in the neural network.

The storage unit 280 stores correspondence information associating each of the plurality of the artificial synapses with a corresponding one of the endocrine substances. The parameter adjusting unit 220 changes the respective coupling coefficients of the plurality of artificial synapses associated with the endocrine substances by the correspondence information, according to the secretion amounts of the endocrine substances indicated by the secretion information generated by the secretion information generating unit 200.

Note that the input information generating unit 210 acquires the measurement values indicated by the detection signals at predetermined frequency, and generates input values that are predetermined values for the neural network. On the other hand, the secretion information generating unit 200 changes secretion amounts of the endocrine substances according to the measurement values indicated by the detection signals. For example, the secretion information generating unit 200 changes the secretion amounts of the endocrine substances according to the magnitude of the measurement values indicated by the detection signals.

The input information generating unit 210 may generate, at different frequencies for the plurality of sensors, input values that are predetermined values for the neural network. For example, in the vehicle 10, input frequency of the input value based on the accelerator opening may be made higher than the input frequency of the input value based on the roll angle. The frequencies may be made different according to kinds of target objects. For example, when the target object is a two-wheel vehicle, input frequency of the input values based on the roll angle may be made higher than when the target object is a four-wheel vehicle. This is because, changes in the roll angle are larger for two-wheel vehicles, so it has more significant influence on the emotion.

Note that the neural network includes a plurality of emotion artificial neurons that are artificial neurons for which emotions are determined. The emotion determining unit 260 determines the current emotion based on respective current firing states of the plurality of emotion artificial neurons. For example, the emotion determining unit 260 may determine, as the emotion of the vehicle 10, an emotion that is assigned to the firing emotion artificial neurons.

The control unit 250 controls the vehicle 10 according to the emotion determined by the emotion determining unit 260. For example, when the emotion of "joyful" is determined by the emotion determining unit 260, the control unit 250 displays a smiling face icon on the UI unit 180. Also, the control unit 250 may output a voice of a bright tone from the UI unit 180 and make a conversation with the user 190. Also, the control unit 250 may display a message of a bright tone on the UI unit 180 and make a conversation with the user 190.

Functions of the units of the emotion determining system 100 may be implemented by a computer. For example, the processing unit 270 may be constructed of a processor etc. such as a MPU, and the storage unit 280 may be constructed of a recording medium such as a non-volatile memory. The storage unit 280 may store a program that is executed by processors. By processors executing the program, the secretion information generating unit 200, the input information generating unit 210, and the parameter adjusting unit 220, the emotion determining unit 260 including the NN operating unit 230 and the emotion judging unit 240, and the control unit 250 are implemented, and control of the storage unit 280 may be carried out. The program may be read out from the recording medium 290 such as an optical disc by the processor and stored in the storage unit 280, or may be provided to the emotion determining system 100 through a network and stored in the storage unit 280. The storage unit 280 and the recording medium 290 may be a computer readable non-transitory recording medium.

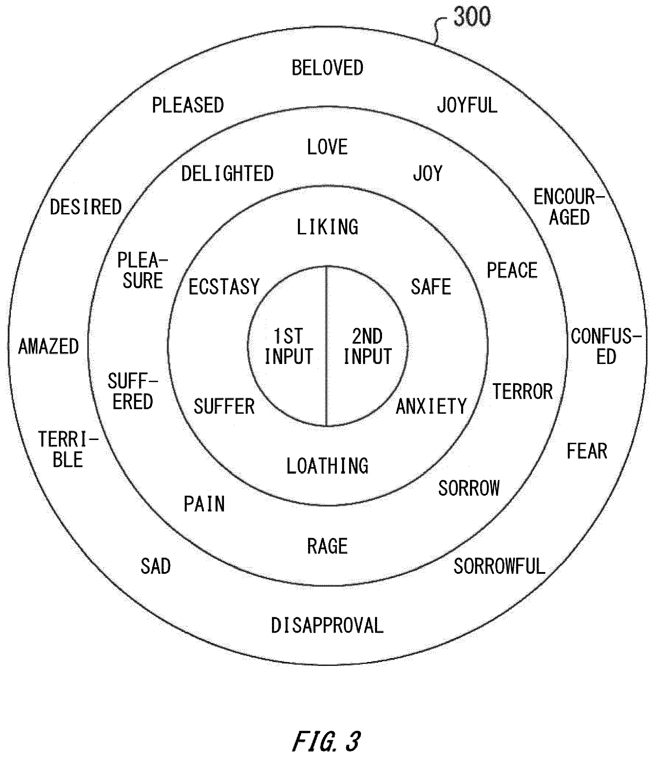

Fig. 3 schematically shows an emotion map 300. In the emotion map 300, emotions are positioned in concentric circles radially from the center. In the closer area to the center of the concentric circle, the more primitive-state-emotions are positioned. In the farther area from the center of the concentric circle, the more emotions indicating states or actions generated from state of minds are positioned. The emotion is a concept also including affectivities and mental states etc. On the left side of the concentric circle, generally, emotions generated from reactions taking place in a brain are positioned. In the right side of the concentric circle, generally, emotions induced by situational judgement are positioned.

The neural network that is an operation object of the NN operating unit 230 includes artificial neurons that are assigned to the respective emotions shown in the emotion map 300. In the neural network, artificial neurons for inputs are respectively assigned also to the first input and the second input that are positioned in the innermost area of the concentric circle in the emotion map 300. The artificial neurons for input that are assigned to the first input and the second input receive input informations based on the detection signals of the sensor unit 110. Then, generally from the inner side to the outer side, the artificial neurons are connected by the artificial synapses and form a neural network. Note that it may be determined, according to a design, whether the input informations based on the detection signals of the sensors of the sensor unit 110 are input to the artificial neuron for input assigned to the first input, input to the artificial neuron for input assigned to the second input, or input to both the artificial neuron for input assigned to the first input and the artificial neuron for input assigned to the second input.

The NN operating unit 230 performs the operation of the neural network repeatedly based on the input information and determines firing states of the respective artificial neurons. From the firing states of the artificial neurons, the emotion judging unit 240 judges the emotion of the vehicle 10. For example, the emotion judging unit 240 judges an emotion to which the firing artificial neuron is assigned as one emotion felt by the vehicle 10.

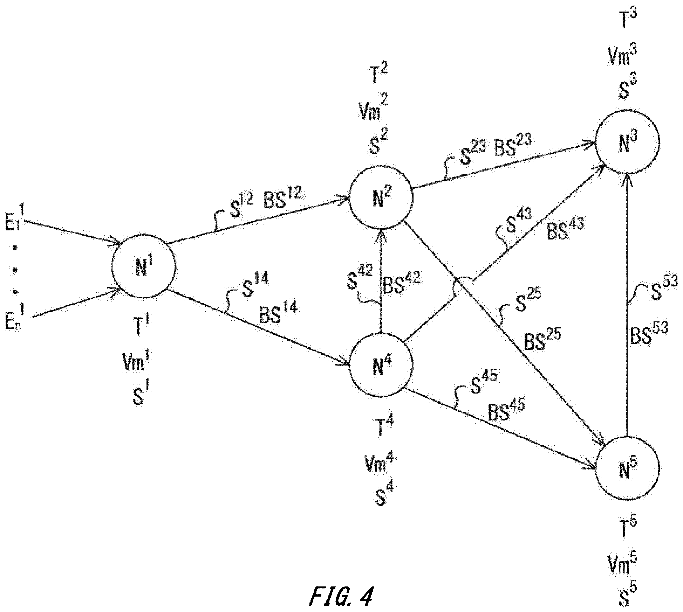

Fig. 4 schematically shows a part of the neural network that is used for the emotion determining system 100. The part of the neural network shown in the figure includes artificial neurons N¹, N², N³, N⁴ and N⁵, and artificial synapses S¹², S¹⁴, S²³, S²⁵, S⁴², S⁴³, S⁴⁵ and S⁵³. Artificial neurons correspond to the neurons in a living body. Artificial synapses correspond to the synapses in a living body.

E¹ indicates input information based on the detection signal. The artificial neuron N¹ is an artificial neuron for input. The artificial neuron N¹ receives n pieces of input information E₁¹ to Eₙ¹ that are generated based on the detection signals of the respective sensors.

The artificial synapse S¹² is an artificial synapse connecting the artificial neuron N¹ and the artificial neuron N². Particularly, the artificial synapse S¹² is an artificial synapse to input the output of the artificial neuron N¹ to the artificial neuron N². The artificial synapse S¹⁴ is an artificial synapse connecting the artificial neuron N¹ and the artificial neuron N⁴. Particularly, the artificial synapse S¹⁴ is an artificial synapse to input the output of the artificial neuron N¹ to the artificial neuron N⁴. Note that an artificial synapse to input the output of the artificial neuron N^{j} to the artificial neuron N^{k} is represented by artificial synapse S^{jk}, where j, k are integer.

Here, each artificial neuron is represented by Nⁱ, where i is integer. Nⁱ has, as the parameters: Sⁱ to represent the status of Nⁱ; Vⁱm to represent the internal state of the artificial neuron represented by Nⁱ; and Tⁱ to represent a threshold for firing of Nⁱ. Also, the artificial synapse S^{jk} has the coupling coefficient BS^{jk} as the parameters. Note that, in the present embodiment, the artificial neurons may be collectively called the artificial neuron N, with the suffix omitted. Also, the artificial synapses may be collectively called the artificial synapse S, with the suffix omitted. Similarly, also the parameters of the artificial neuron may be collectively called the internal information Vm, the threshold T, and the status S, with those suffixes omitted.

The status S of the artificial neuron N, the internal state Vm, and the threshold T are parameters that may be updated as time elapses. The status S is information related to the firing state of the neuron, and indicates at least whether the artificial neuron is in a firing state or in a non-firing state. The internal state Vm is information related to membrane potential of the neuron, and an exemplary parameter indicating an internal state or an output of the artificial neuron N.

Also, the coupling coefficient BS that is a parameter of the artificial synapse S is a parameter that may be updated as time elapses. The coupling coefficient BS is information related to synaptic plasticity, and indicates the strength of coupling between the artificial neurons N which are coupled via the artificial synapse S.

The NN operating unit 230 updates, from the input information, the parameters described above in the neural network and calculates the internal state Vm of each artificial neuron N. Note that, in the present embodiment, when the internal state Vm exceeds the threshold T, the artificial neuron N turns the status S into the "firing" state. When turned in the firing state, the artificial neuron N outputs a predetermined signal for predetermined time. Once the predetermined time has elapsed, the status S of N returns to a non-firing state.

Here, contents of the operations by the NN operating unit 230 are more specifically described with N² taken as an example. The NN operating unit 230 calculates the input I² to N² by the expression: BS¹²×Vm¹×f(S¹)+BS⁴²×Vm⁴×f(S⁴). Here, f(s) is a function that returns 0 when S is a value indicating non-firing state and returns 1 when S is a value indicating an increasing or decreasing phase. Note that this f(s) corresponds to a model where synapses transmit action potential only when neurons fire. Note that the relationship: f(S)=1 may be satisfied. This corresponds to a model where the membrane potential is transmitted regardless of the firing state of neurons. For f(S), functions corresponding to other transmission models of the membrane potential may be applied.

Generally, the NN operating unit 230 calculates the input Iⁱ to Nⁱ by the expression: ∑ⱼBS^{ji}×Vm^{j}×f(S^{j})+∑ⱼEⱼⁱ. The NN operating unit 230 uses BS^{ji}, Vm^{j}, S^{j}, E^{j} at the present timing and calculates the input Iⁱ, Sⁱ and the like to Nⁱ for the next timing. The NN operating unit 230, by repeating this in time, determines the status S of each artificial neuron N in real-time. Then, the emotion judging unit 240 judges the emotion of the vehicle 10 based on the status S of each artificial neuron N. For example, when the artificial neuron assigned to the emotion of "joyful" in Fig. 3 is fired, the emotion judging unit 240 can judge that the vehicle 10 has an emotion of "joyful".

Here, the secretion information generating unit 200 adjusts BS based on the detection signals of the sensor unit 110. For example, when the accelerator opening is detected as 100% by the accelerator opening sensor of the sensor unit 110, the secretion information generating unit 200 increases, as the internal variables, the secretion amount of "noradrenaline" and the secretion amount of "dopamine". Then, based on the secretion amount of "noradrenaline" and the secretion amount of "dopamine", the coupling coefficient BS of the artificial synapse S associated with at least one of "noradrenaline" and "dopamine" is adjusted.

The sensors of the sensor unit 110 are each associated with particular endocrine substances, and the secretion amounts of the internal secretion substance are associated with the coupling coefficients BS of the particular artificial synapses S. Thereby, detection signal of the sensor unit 110, via the secretion amount of the internal secretion substance, can change the easiness of the signal transmission in the artificial synapse S at each part in the neural network. This enables to generate a variety of emotions of the detection signals detected by the sensor unit 110.

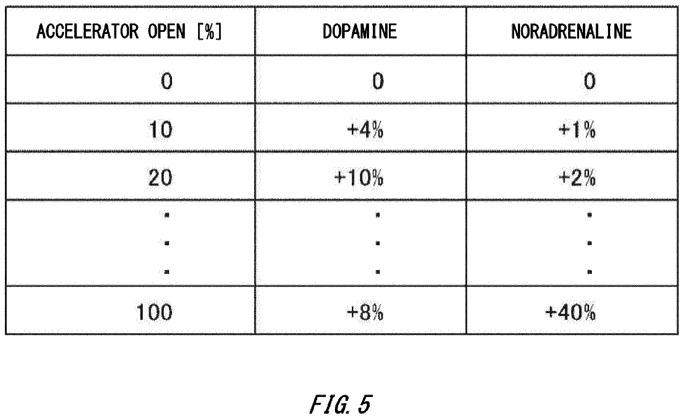

Fig. 5 is exemplary sensor correspondence information associating the accelerator opening with the amounts of the endocrine substances. The storage unit 280 stores, associated with a plurality of values of the accelerator openings, informations indicating dopamine and noradrenaline. More specifically, the storage unit 280 stores, associated with the respective accelerator openings, information indicating combination of the increased amount in the secretion amount of dopamine and the increased amount in the secretion amount of the noradrenaline. Note that the increased amounts in the secretion amounts are indicated by the percentage to the upper limit value of the secretion amounts represented by the internal variables used by the NN operating unit 230.

Stepping in the accelerator device means an intention to drive the vehicle 10. Compared to a human being, this corresponds to that a human being intends to run. When human beings run, noradrenaline is secreted and sugar is carried into the blood. Also, when human beings take exercise, dopamine is secreted. Thus, the step-in quantity of the accelerator device in the vehicle 10 is associated with secretion of noradrenaline and dopamine.

Note that the noradrenaline is, in addition to preventing or reducing decrease in the blood sugar level, involved in sense of anxiety and sense of fear. Thus, as shown in the figure, it is preferable that the greater accelerator opening is, the more secretion increase amount of noradrenaline is. This is because the greater accelerator opening results in the higher driving speed of the vehicle 10, which help to cause sense of anxiety and sense of fear to be felt. On the other hand, regarding dopamine, less secretion increase amount than that for the accelerator opening of 20% is associated with the accelerator opening of 100%. For human beings, dopamine is involved in sense of happiness, and, when the accelerator opening is extremely great, the secretion increase amount of dopamine is desired to be reduced. For example, when the accelerator opening is less than 100%, the secretion increase amount of dopamine is preferably made to have a maximum value.

Note that, according to the correspondence information shown in the figure, dopamine and noradrenaline are associated with the accelerator opening. However, the secretion increase amounts of more number of endocrine substances may be associated with the accelerator opening.

Note that the secretion increase amount associated with the accelerator opening may be different for different kinds of target objects. For example, when the target object is two-wheel vehicle, more secretion increase amount of noradrenaline may be associated than when the target object is a four-wheel vehicle. Also, when the target object is two-wheel vehicle, less secretion increase amount of dopamine may be associated with than when the target object is a four-wheel vehicle. This is because, when driving a two-wheel vehicle, the impact on a human body at an accident is more serious than when driving a four-wheel vehicle, and thus it is considered that more sense of fear and/or less sense of happiness is/are felt when the accelerator opening is greater.

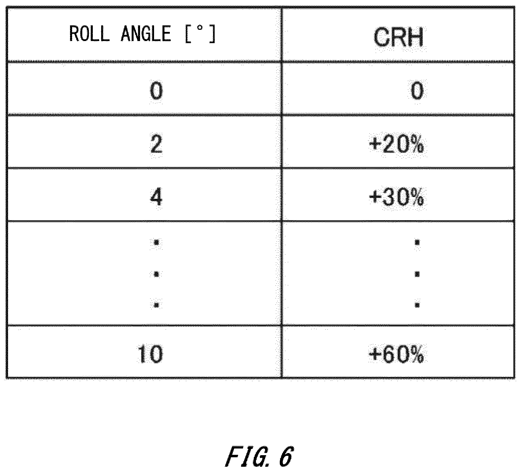

Fig. 6 is exemplary sensor correspondence information associating the roll angle with the endocrine substances. The storage unit 280 stores information indicating CRH, associated with the roll angle. More specifically, the storage unit 280 stores information indicating increased amount in the secretion amount of CRH, associated with a plurality of values of the roll angle

That the roll angle of the vehicle 10 gets big corresponds to, compared to a human being, that the body of the human being is leaned. When the body is leaned, the secretion of CRH is stimulated for protection against stress. Thus, the roll angle is associated with secretion of CRH. Specifically, the greater the roll angle is, the greater value as the secretion increase amount of CRH is associated. This is because the greater roll angle is considered to cause the greater stress.

Note that, according to correspondence information indicating the figure, only CRH is associated with the roll angle. However, the secretion increase amounts of more number of endocrine substances may be associated with the roll angle.

Note that the secretion increase amount associated with the roll angle may be different for different kinds of target objects. For example, when the target object is two-wheel vehicle, more secretion increase amount of CRH may be associated than when the target object is a four-wheel vehicle. This is because, when driving a two-wheel vehicle, the greater roll angle results in the higher risk of slipping than when driving a four-wheel vehicle and thus it is considered that more sense of fear is felt when the roll angle is greater.

In Figs. 5 and 6, it has been described that the measurement values of the accelerator opening and the roll angle are associated with the secretion increase amounts of the internal secretion substances. However, measurement values of other arbitrary sensors may be associated with the secretion increase amounts of the internal secretion substances. Also, to simplify the description, the measurement values of the accelerator opening and the roll angle and the secretion increase amounts of the internal secretion substances are represented by the discrete values, but the secretion increase amounts of the internal secretion substances may be associated by continuous functions etc. whose variables are the measurement values such that continuous secretion increase amounts of the internal secretion substances can be obtained for continuous changes in the measurement values. Note that, as the sensor correspondence information, as an alternative to associating the measurement values with the secretion increase amounts of the internal secretion substances, or, in addition to associating the measurement values with the secretion increase amounts of the internal secretion substances, the change amounts over time of the measurement values may be associated with the secretion increase amounts of the internal secretion substances.

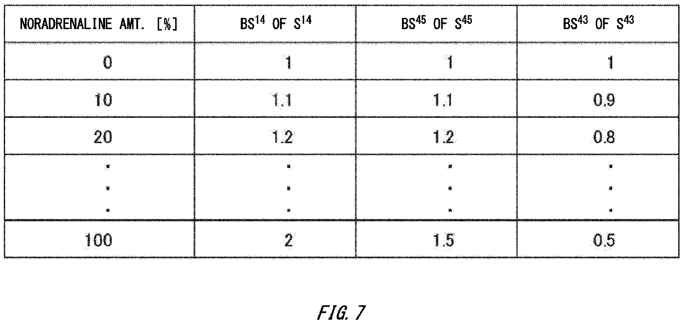

Fig. 7 is exemplary coupling coefficient correspondence information associating the noradrenaline amount with the coupling coefficients BS. The storage unit 280 stores information to associate, with the total secretion amount of noradrenaline, the increasing coefficient of coupling coefficient BS¹⁴ of artificial synapse S¹⁴, the increasing coefficient of coupling coefficient BS⁴⁵ of artificial synapse S⁴⁵, and the increasing coefficient of coupling coefficient BS⁴³ of artificial synapse S⁴³. Note that the artificial synapse S mentioned here connects the artificial neurons N with strong coupling.

As shown in the figure, the greater the noradrenaline amount is, the greater values are associated with the increasing coefficient of BS¹⁴ and the increasing coefficient of BS⁴⁵. On the other hand, the greater the noradrenaline amount is, the smaller value is associated with the increasing coefficient of BS⁴³. Thereby, for example, in the neural network shown in Fig. 4, signals generated by the input information are transmitted more easily in a direction from N¹ toward N⁵, than in a direction from N¹ toward N³. Thus, artificial neurons positioned in the direction from N¹ toward N⁵ are fired more easily. Thus, for example, in the emotion map shown in Fig. 3, as noradrenaline gets increased, emotions that are positioned in a particular direction with respect to the center portion of the concentric circle, for example, emotions such as "anxiety" and "fear" become easy to fire. This can help emotions that is similar to the emotions generated in human beings to be generated in the vehicle 10.

Note that it has been described here that the coupling coefficient BS of the artificial synapse S is adjusted in a direction to make the artificial neuron N at an output destination easier to fire. However, the increasing coefficient may be set such that the coupling coefficient BS of the artificial synapse S can be adjusted in a direction to make the artificial neuron N at the output destination harder to fire. For example, when the artificial synapse S is strong coupling, making the increasing coefficient small enables to make the artificial neuron N at the output destination harder to fire. Note that, when the artificial synapse S connects the artificial neurons N with inhibitory coupling, making the increasing coefficient large enables to make the artificial neuron N at the output destination harder to fire, whereas making the increasing coefficient small enables to make the artificial neuron N at the output destination easier to fire.

The parameter adjusting unit 220 refers to the coupling coefficient correspondence information and adjusts corresponding coupling coefficients BS by the amount according to the total secretion amounts of the internal secretion substances. As described above, the secretion information generating unit 200 determines the total secretion amount of the respective internal secretion substances according to the measurement values of the respective sensors. Thus, using the measurement values of the sensors, complex adjustment of the amount of the coupling coefficient BS is possible, and then, the emotion artificial neuron can be fired in a variety of combinations. Furthermore, by associating, based on comparison to a human being, the sensors in the vehicle 10 with the corresponding internal secretion substances, and the internal secretion substances with the corresponding coupling coefficients BS, emotions causing no sense of strangeness for human beings can be generated.

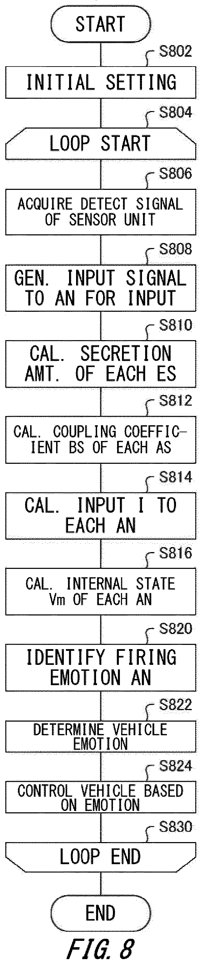

Fig. 8 is a flowchart illustrating operations of the units in the emotion determining system 100. When starting emotion generation processing is directed, in step 802, the NN operating unit 230 performs initial setting for parameters in the neural network. For example, the NN operating unit 230 reads out initial values for the parameters from the storage unit 280 and initializes the parameters in the neural network (step 802). Upon completion of the initial setting, in step 804, processing loop per timing starts.

In step 806, the input information generating unit 210 and the secretion information generating unit 200 acquire detection signals of the sensor unit 110. In step 808, the input information generating unit 210 generates input information to the artificial neuron for input that is assigned to the first input and input information to the artificial neuron for input that is assigned to the second input. The input information generating unit 210 generates an input pulse of a constant value that is generated at a prescribed sampling interval where the detection signals are acquired from the respective sensors as the input information.

In step 810, the secretion information generating unit 200 calculates the secretion amount of the endocrine substance, based on the sensor correspondence information described in connection with e.g. Figs. 5, 6 and the like, the measurement values of the detection signals of the sensors that are acquired in step 806. Then, in step 812, the parameter adjusting unit 220 calculates the coupling coefficients BS of the artificial synapses S.

Then, in step 814, the NN operating unit 230 calculates inputs I to the artificial neurons by the expression described in connection with Fig. 4 and the like. Then, in step 816, the NN operating unit 230 calculates the internal states Vm of the artificial neurons, based on the inputs I to the artificial neurons.

Then, in step 820, the NN operating unit 230 determines firing emotion artificial neurons based on the internal states Vm of the emotion artificial neurons and the thresholds T. In step 822, the emotion judging unit 240 judges the emotion of the vehicle 10 based on the firing emotion artificial neurons. Thereby, the emotion determining unit 260 assigns, as the emotion of the vehicle 10, emotions corresponding to the firing emotion artificial neurons. Note that the emotion judging unit 240 may judge that the vehicle 10 more strongly feels an emotion corresponding to an emotion artificial neuron having the internal states Vm of a bigger value among the firing emotion artificial neurons. Then, in step 824, the control unit 250 controls the respective units of the vehicle 10 based on the emotion judged in step 822.

In S830, the emotion determining system 100 judges whether to terminate the loop or not. For example, when terminating the emotion generation processing is directed, the loop is judged to end. When the loop is not to be terminated, the process returns to 804, and calculation for the still next time step is performed.

In S830, the emotion determining system 100 judges whether to terminate the loop or not.

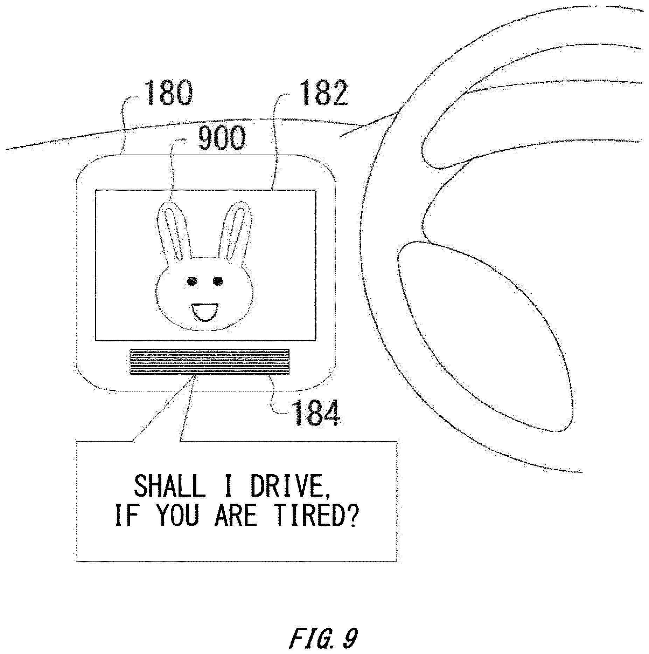

Fig. 9 schematically shows information output from the UI unit 180. For example, suppose that the emotion determining unit 260 determines that the vehicle 10 has emotions of "beloved", "sad", and "anxiety". In this case, the control unit 250 displays, on a display unit 182 being a navigation apparatus as one example of the UI unit 180, an object 900 having an expression associated with the determined emotion. Also, the control unit 250 outputs a voice associated with the determined emotion and the current situation of the vehicle 10 from a voice outputting unit 184.

Thereby, the driver can feel as if being able to share pleasure and pain with the vehicle 10. As such, the driver can feel as if sharing the emotion with the vehicle 10.

Also, for example, when a driver who is usually not good at operation of the steering device drives through a sharp turn with smooth operation of the steering device, "pleasure" is determined for the emotion of the vehicle 10, an expression indicating feeling of pleasure is displayed on the display unit 182. Thereby, the driver feels better and feels encouraged more easily to drive the vehicle 10 better. Thus, expressing the information based on the sensor information as the emotion of the vehicle 10 may encourage the driver to improve the driving technique.

Note that, in the above description, as described in connection with Fig. 7 and the like, for example, the coupling coefficient BS of the artificial synapse S has been mainly taken as the adjusting parameter and described. However, the adjusting parameter is not limited to the coupling coefficient BS of the artificial synapse S. For example, the adjusting parameters can include the threshold T of the artificial neuron N, the output value from the artificial neuron N when the artificial neuron N fires, and the like.

Also, the function of emotion determining system 100 may be implemented by a plurality of computers. For example, part of the function of the emotion determining system 100 may be implemented by computers provided in the vehicle 10, and the other function of the emotion determining system 100 may be implemented by one or more computers provided outside of the vehicle 10 which communicate with the computers provided in the vehicle 10 via communication network. The function of the one or more computers provided outside the vehicle 10 may be implemented in the cloud.

The vehicle 10 is not limited to a four-wheel vehicle, and may be various automobiles such as a two-wheel vehicle. The vehicle 10 may be the electric vehicle, the hybrid vehicle, or the like, which includes the electric motor as at least part of the power. Note that the vehicle 10 is one example of the system including the emotion determining system. For the system including the emotion determining system, various forms other than vehicles, can be applied. The systems including the emotion determining system can include various mobilities other than vehicles, robots, electric equipment, buildings, etc.

While the embodiments of the present invention have been described, the technical scope of the invention is not limited to the above described embodiments. It is apparent to persons skilled in the art that various alterations and improvements can be added to the above-described embodiments. It is also apparent from the scope of the claims that the embodiments added with such alterations or improvements can be included in the technical scope of the invention.

The operations, procedures, steps, and stages of each process performed by an apparatus, system, program, and method shown in the claims, embodiments, or diagrams can be performed in any order as long as the order is not indicated by "prior to," "before," or the like and as long as the output from a previous process is not used in a later process. Even if the process flow is described using phrases such as "first" or "next" in the claims, embodiments, or diagrams, it does not necessarily mean that the process must be performed in this order.

### EXPLANATION OF REFERENCES

10: vehicle; 100: emotion determining system; 110: sensor unit; 180: UI unit; 190: user; 200: secretion information generating unit; 210: input information generating unit; 220: parameter adjusting unit; 230: NN operating unit; 240: emotion judging unit; 250: control unit; 260: emotion determining unit; 270: processing unit; 280: storage unit; 290: recording medium; 300: emotion map; 182: display unit; 184: voice outputting unit; 190: user; 900: object

## Claims

1. An emotion determining system comprising:
an input information generating unit to generate, based on detection signals of one or more sensors that are provided to a target object, input information for determining an emotion of the target object;
a secretion information generating unit to generate, based on the detection signals, secretion information indicating secretion amounts of one or more endocrine substances;
a parameter adjusting unit to adjust an operation parameter for determining the emotion from the input information based on the secretion amounts of the one or more endocrine substances indicated by the secretion information.

2. The emotion determining system according to claim 1, further comprising
a first correspondence information storage unit to store first correspondence information that associates a plurality of the sensors with the secretion amounts of the one or more endocrine substances respectively, wherein
the secretion information generating unit changes the secretion amounts of the one or more endocrine substances associated with the respective sensors by the first correspondence information according to measurement values indicated by the detection signals of the plurality of sensors.

3. The emotion determining system according to claim 1 or 2, further comprising:
an emotion determining unit to determine the emotion from the input information using the operation parameter.

4. The emotion determining system according to claim 3, wherein
the emotion determining unit determines the emotion using a neural network that receives the input information as an input.

5. The emotion determining system according to claim 4, wherein
the operation parameter is a coupling coefficient of an artificial synapse that is included in the neural network.

6. The emotion determining system according to claim 5, further comprising a second correspondence information storage unit to store second correspondence information that associates each of a plurality of the artificial synapses with a corresponding one of the endocrine substances, wherein
the parameter adjusting unit changes each of the coupling coefficients of the plurality of artificial synapses associated with the endocrine substances by the second correspondence information according to the secretion amounts of the one or more endocrine substances indicated by the secretion information.

7. The emotion determining system according to any one of claims 4 to 6, wherein
the input information generating unit acquires measurement values indicated by the detection signals at predetermined frequency and generates an input value that is a predetermined value for the neural network, and
the secretion information generating unit changes the secretion amounts of the one or more endocrine substances according to the measurement values.

8. The emotion determining system according to claim 7, wherein
the input information generating unit generates input values that are predetermined values for the neural network at the frequencies that are different for a plurality of the sensors.

9. The emotion determining system according to any one of claims 4 to 8, wherein
the neural network includes a plurality of emotion artificial neurons that are artificial neurons, for the artificial neurons emotions being determined, and
the emotion determining unit determines the emotion of present time based on respective current firing states of the plurality of emotion artificial neurons.

10. The emotion determining system according to any one of claims 1 to 9, wherein
the secretion information generating unit changes the secretion amounts of the one or more endocrine substances according to change amounts over time of measurement values indicated by the detection signals.

11. The emotion determining system according to any one of claims 1 to 10, further comprising
a control unit to control the target object according to the emotion.

12. A system comprising:
the emotion determining system according to any one of claims 1 to 11; and
the target object.

13. A program to make a computer to function as the emotion determining system according to any one of claims 1 to 11.
